(19) Europäisches Patentamt
European Patent Office
Office européen des brevets

(11) EP 1 743 628 A1

(12) DEMANDE DE BREVET EUROPEEN

(43) Date de publication:
**17.01.2007 Bulletin 2007/03**

(51) Int Cl.:
***A61K 8/97*** (2006.01)

(21) Numéro de dépôt: **06117159.1**

(22) Date de dépôt: **13.07.2006**

(84) Etats contractants désignés:
**AT BE BG CH CY CZ DE DK EE ES FI FR GB GR HU IE IS IT LI LT LU LV MC NL PL PT RO SE SI SK TR**
Etats d'extension désignés:
**AL BA HR MK YU**

(30) Priorité: **13.07.2005 FR 0507520**

(71) Demandeurs:
- **Simer**
**29480 Le Relecq Kerhuon (FR)**
- **Université de Bretagne Occidentale**
**29269 Brest (FR)**

(72) Inventeurs:
- **Deslandes, Eric**
**29217 Plougonvelin (FR)**
- **Bodeau, Christine**
**29200 Brest (FR)**

(74) Mandataire: **Ahner, Francis et al**
**Cabinet Régimbeau**
**20, rue de Chazelles**
**75847 Paris cedex 17 (FR)**

(54) **Composition cosmétique comprenant un extrait d'algue rouge comprenant une association de floridoside et d'acide iséthionique.**

(57) La présente invention concerne une composition cosmétique comprenant en tant que principe actif une association de floridoside et d'acide iséthionique, dans un rapport massique compris entre 1 et 5. Ce principe actif peut être un extrait d'algue rouge.

L'invention concerne également l'utilisation de cette composition cosmétique pour hydrater la peau et/ou pour prévenir le vieillissement cutané. On décrit également une méthode de traitement cosmétique des peaux déshydratées et/ou sèches et une méthode de traitement cosmétique des peaux âgées et/ou ridées.

EP 1 743 628 A1

## Description

L'invention concerne une composition cosmétique comprenant en tant que principe actif un extrait d'algue rouge comprenant une association de floridoside et d'acide iséthionique et un véhicule cosmétiquement acceptable.

Le floridoside (2-O-glycérol-α-D-galactopyranoside) constitue la principale source de réserve de carbone soluble chez les Rhodophycées (Bean et Hassid, 1955 ; Kremer et Kirst, 1982 ; Wu et Gretz, 1993) sauf chez les céramiales où il est remplacé par le diginéaside (2-O-acide glycérique-D-mannopyranoside) (Reed 1990).

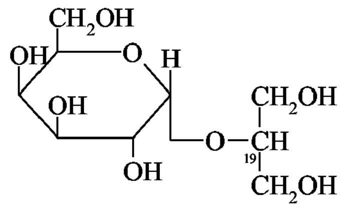

Floridoside

Le Floridoside et l'acide iséthionique (Simon-Colin et al., 2002; Broberg et al., 1998) sont des osmolytes que l'on retrouve dans certaines algues rouges. Les osmolytes sont des solutés organiques accumulés en réponse à un stress osmotique pour rétablir une pression intracellulaire cytoplasmique favorable à la croissance. Dans les algues rouges, les teneurs en floridosides sont généralement comprises entre 1,5 et 8% en poids de la matière sèche mais peuvent atteindre 22% chez *Catenella nipae* (Kirst, 1980 ; Simon-Colin et al., 2002). En plus du floridoside, certaines Rhodophycées appartenant aux Bangiales et plus particulièrement aux genres *Porphyra* et *Bangia* possèdent deux formes isomériques du floridoside : le D-isofloridoside (α-D-galactopyranosyl-(1-1)-D-glycérol) et le L-isofloridoside (α-D-galactopyranosyl-(1-1)-L-glycérol) (Meng et al, 1987 ; Karsten et al, 1991,1993); ces trois hétérosides pouvant représenter jusqu'à 30% du poids sec de l'algue. La principale fonction actuellement reconnue pour le floridoside est son intervention dans l'ajustement osmotique notamment dans la résistance aux stress hyperosmotiques aux cours desquels sa teneur augmente fortement (Reed et al., 1980 ; Karsten et al., 1993). C'est également l'un des produits majeurs accumulés au cours de la photosynthèse et il constitue la principale source de carbone soluble, qui sera ensuite utilisée selon les besoins de la cellule (Yu, 1992 ; Macler, 1986, Goulard F. et al, 2001).

Enveloppe de protection et d'échanges avec l'environnement, la peau est un organe à part entière, jouant un rôle fondamental dans la santé, le confort et l'apparence extérieure. On distingue deux régions formant la peau: l'épiderme (tissu épithélial) et le derme (tissu conjonctif). Les kératinocytes sont les cellules majoritaires de l'épiderme. Elles synthétisent de la kératine, une protéine fibreuse qui donne de la dureté à l'épiderme et le protège contre la déshydratation. Avec le temps, la peau perd de sa souplesse et sa capacité à retenir l'eau diminue. La déshydratation contribue ainsi au vieillissement cutané. L'hydratation de la peau lui permet de préserver sa souplesse, sa douceur, sa tonicité et son aspect. Le stratum corneum est la zone la plus superficielle de l'épiderme. Elle est constituée d'un empilement de cellules plates et anucléées, les cornéocytes, résultat ultime du processus de différenciation et de prolifération des kératinocytes. Les cornéocytes sont liés entre eux par un ciment lipidique et des jonctions intercellulaires, les cornéodesmosomes, assurant la cohésion du système. Le stratum corneum, par sa solidité et grâce à sa structure stratifiée compacte, assure une fonction barrière : il s'oppose à la perte en eau transcutanée et protège les couches sous-jacentes des agressions mécaniques, chimiques et de l'irradiation ultra violet (UV). Le stratum corneum est capable de capter et retenir l'eau non liée (hydratation supplémentaire) qui se trouve principalement dans les espaces intercellulaires. La quantité d'eau contenue dans la couche cornée intervient dans la qualité de nombreuses fonctions de la peau : propriétés mécaniques et transparence du stratum corneum, et donc de la peau dans sa globalité, fonction barrière (absorption/perte d'eau, protection vis à vis d'agression extérieures), desquamation.

De nombreuses compositions cosmétiques sont proposées pour hydrater la peau et ainsi prévenir le vieillissement cutané intrinsèque ou extrinsèque (soleil, tabac, pollution, stress, ménopause). L'homme de l'art est toujours à la recherche de nouvelles compositions qui présentent une activité anti-déshydratante et qui permettent de protéger les kératinocytes.

D'une manière surprenante, les inventeurs ont découvert qu'une composition comprenant en tant que principe actif une association de floridoside et d'acide iséthionique présente une activité anti-déshydratante et permet de protéger les kératinocytes. Le floridoside et l'acide iséthionique peuvent être extraits de certaines algues rouges.

La demande de brevet FR 2 655 268 décrit l'utilisation d'extraits d'algues dans des compositions cosmétiques, pharmaceutiques, alimentaires ou à usage agricole, à activité anti-radicalaire. Les extraits d'algues sont obtenus par un procédé simple, qui ne comprend pas d'étape d'ultrafiltration ou de nanofiltration.

La demande internationale WO 03/041679 décrit des compositions cosmétiques comprenant un extrait d'algue rouge (genre Porphyra). Ces compositions cosmétiques permettent d'induire la synthèse des protéines de stress et sont donc utiles pour la prévention du vieillissement cutané. Toutefois, l'algue rouge Porphyra ne contient pas d'acide iséthionique.

Ainsi, l'invention a pour objet une composition cosmétique comprenant en tant que principe actif une association de floridoside et d'acide iséthionique, dans un rapport massique compris entre 1 et 5, et un véhicule cosmétiquement acceptable,

Dans la composition cosmétique selon l'invention, le rapport massique floridoside/acide iséthionique est avantageusement compris entre 1,5 et 4, plus avantageusement compris entre 2 et 3. La composition cosmétique comprend avantageusement 0,01 à 20% en poids, plus avantageusement 0,03 à 10% en poids de la combinaison de floridoside et d'acide iséthionique, par rapport au poids total de la composition.

Le principe actif utilisé dans la composition selon l'invention est un extrait d'algue rouge, qui comprend une association de floridoside et d'acide iséthionique, dans un rapport massique compris entre 1 et 5. Cet extrait d'algue rouge comprend, en poids par rapport au poids total de la matière sèche de l'extrait :

- Floridoside 25% - 50%
- Acide iséthionique 10% - 25%

Les autres constituants majoritaires dudit extrait sont avantageusement des protéines (20 à 35 %) et les glucides (5 à 20 %). La matière sèche est constituée de la matière organique et minérale de l'extrait, c'est ce qui reste après évaporation totale de l'eau présente dans l'extrait. Son poids est déterminé par différence entre le poids de l'extrait avant et après un séjour en étuve jusqu'à dessiccation totale. La teneur en matière sèche dans l'extrait d'algue rouge selon l'invention est avantageusement de 0,5 à 20% en poids, plus avantageusement de 1 à 10% en poids, par rapport au poids total de l'extrait.

La source commune de floridoside et d'acide iséthionique est un extrait d'algue rouge qui appartient avantageusement à la famille des *Gigartinaceae,* plus avantageusement au genre *Mastocarpus,* encore plus avantageusement à l'espèce *Mastocarpus stellatus.*

Selon une variante de l'invention, la composition cosmétique comprend avantageusement 10 à 100% en poids, plus avantageusement 50 à 100% en poids d'un extrait selon l'invention, par rapport au poids total de la composition.

Selon une autre variante de l'invention, la composition cosmétique comprend avantageusement 0,05 à 10% en poids, plus avantageusement 0,1 à 5% en poids d'un extrait selon l'invention, par rapport au poids total de la composition.

La composition cosmétique peut en outre comprendre un extrait peptidique de spiruline, tel que décrit dans la demande de brevet FR 2 857 978 par exemple. Cet extrait peptidique de spiruline comprend avantageusement 70 à 80 % en poids de peptides, 4 à 5 % en poids de sucres et 1% en poids de chlorure de sodium, par rapport au poids total de l'extrait. Dans cet extrait, les peptides présentent avantageusement la même composition en acides aminés, qualitativement et au moins sensiblement quantitativement, que la microalgue spiruline. Avantageusement, au moins 70 % des peptides ont un degré de polymérisation inférieur à 10 et présentent une masse molaire inférieure à 1 250 Da. L'extrait peptidique de spiruline peut être obtenu par un procédé comprenant au moins les étapes successives suivantes :

a) séparation des lipides et des protéines de la spiruline en soumettant des microalgues de spiruline, sous forme de poudre séchée, à une extraction de la phase lipidique au moyen d'un solvant d'extraction approprié, choisi parmi les solvants polaires et les huiles de synthèse ou végétales, et récupération de la phase lipidique d'une part et de la phase contenant les protéines d'autre part ; puis

b) la phase contenant les protéines issue de l'étape a) est soumise à une hydrolyse enzymatique, qui conduit à l'obtention d'un extrait peptidique; et

c) éventuellement, purification de l'extrait peptidique obtenu suite à l'étape b).

La composition cosmétique comprend avantageusement 0,05 à 20% en poids, plus avantageusement 0,05 à 10% en poids, encore plus avantageusement 0,5 à 10% en poids d'un extrait peptidique de spiruline (tel que décrit précédemment), par rapport au poids total de la composition.

La composition cosmétique peut en outre comprendre de l'eau de mer. L'eau de mer a une teneur en eau en moyenne de 97% (en poids, par rapport au poids total de l'eau de mer). Les éléments majoritairement présents dans l'eau de mer sont avantageusement le chlore, le sodium, le magnésium, le soufre, la calcium, le potassium, le brome, le carbone, le strontium, le bore, le silicium et le fluor. L'eau de mer peut également comprendre les éléments minoritaires suivants : le lithium, le rubidium, le phosphore, l'iode, le baryum, le zinc, le molybdène, l'aluminium, le fer, l'arsenic, l'uranium, le cuivre, le vanadium, le nickel, le manganèse et le titane. L'eau de mer peut également comprendre, à l'état de traces, les éléments suivants : l'étain, le césium, le sélénium, l'yttrium, le cobalt, le tungstène, le cadmium, le chrome,

le scandium, l'argent, le mercure, le plomb et l'or.

Le ratio massique extrait d'algue rouge selon l'invention/extrait peptidique de spiruline/eau de mer dans la composition cosmétique est avantageusement de 50/50/0, plus avantageusement de 25/25/30, encore plus avantageusement de 25/25/13.

La composition cosmétique selon l'invention comprend avantageusement de 0,1 à 30% en poids, plus avantageusement de 0,5 à 20% en poids, encore plus avantageusement de 0,5 à 10% en poids de l'association extrait d'algue rouge, extrait peptidique de spiruline et eau de mer. Les pourcentages massiques sont exprimés par rapport au poids total de la composition cosmétique.

La composition cosmétique peut se présenter sous toutes les formes galéniques normalement utilisées pour une application topique, notamment sous forme d'une solution aqueuse, hydroalcoolique ou huileuse, d'une émulsion huile-dans-eau ou eau-dans-huile ou multiple, d'un gel aqueux ou huileux, d'un produit anhydre liquide, pâteux ou solide, d'une dispersion d'huile dans une phase aqueuse à l'aide de sphérules, ces sphérules (nanoparticules polymérique, vésicules lipidiques de type ionique et ou non ionique), d'un dispositif trans-dermique ou sous toute autre forme pour application topique. Cette composition peut être plus ou moins fluide et avoir l'aspect d'une crème, d'une pommade, d'un lait, d'une lotion, d'un sérum, d'une pâte, d'une mousse ou d'un gel. Elle peut éventuellement être appliquée sur la peau sous forme d'aérosol ou se présenter sous une forme solide, et par exemple sous forme de stick. Elle peut aussi être appliquée au moyen d'un patch.

La composition selon l'invention peut contenir également les adjuvants habituels dans le domaine cosmétique, tels que les gélifiants hydrophiles ou lipophiles, les actifs hydrophiles ou lipophiles, les épaississants, les conservateurs, les antioxydants, les solvants, les parfums, les agents chélateurs, les absorbeurs d'odeur, des filtres chimiques ou minéraux, des pigments minéraux, les tensioactifs, les polymères, les huiles de silicone et les matières colorantes. Les quantités de ces différents adjuvants sont celles classiquement utilisées dans les domaines considérés, et par exemple de 0,01 à 20% du poids total de la composition.

Les modes d'administration, les posologies et les formes galéniques optimales des compositions selon l'invention peuvent être déterminés selon les critères généralement pris en compte dans l'établissement d'un traitement cosmétique, de préférence dermatologique, adapté à un patient comme par exemple le degré d'hydratation de la peau du patient, la tolérance au traitement, le type de peau.

La composition cosmétique selon l'invention présente avantageusement une activité anti-déshydratante. Elle présente également une activité protectrice des kératinocytes (protection contre la déshydratation et protection contre les agressions). La composition cosmétique selon l'invention permet d'augmenter significativement la capacité de rétention d'eau du stratum corneum. La composition cosmétique selon l'invention permet en plus de protéger les cellules, notamment la peau, des agressions externes, telles que le froid, la chaleur, le rayonnement solaire (notamment le rayonnement ultra violet), le vent. La composition cosmétique permet donc de prévenir et/ou retarder le vieillissement cutané, notamment le vieillissement cutané extrinsèque.

Selon une variante de l'invention, la composition est utilisée pour son activité anti-déshydratante. Elle peut alors comprendre des quantités importantes d'extrait d'algue rouge selon l'invention. Selon cette variante, la composition cosmétique selon l'invention comprend avantageusement 10 à 100 % en poids dudit extrait, plus avantageusement 50 à 100 % en poids dudit extrait par rapport au poids total de la composition.

Selon une autre variante de l'invention, la composition est utilisée pour son activité sur les protéines de stress. Elle comprend alors avantageusement 0,1 à 10 % en poids d'extrait d'algue rouge selon l'invention, plus avantageusement, 0,1 à 5 % en poids dudit extrait par rapport au poids total de la composition.

L'invention concerne aussi une méthode de traitement cosmétique des peaux déshydratées et/ou sèches, caractérisée en ce qu'elle comprend une étape d'application topique sur la peau d'une composition cosmétique selon l'invention, telle que décrit précédemment.

L'invention concerne également une méthode de traitement cosmétique des peaux âgées et/ou ridées, caractérisée en ce qu'elle comprend une étape d'application topique sur la peau d'une composition cosmétique selon l'invention, telle que décrite précédemment. Le vieillissement cutané peut être intrinsèque ou d'origine extrinsèque (soleil, tabac, pollution, stress, vent, froid).

L'invention concerne aussi l'utilisation d'une composition cosmétique selon l'invention, telle que décrit précédemment pour hydrater la peau et/ou protéger la peau des agressions extérieures.

L'invention concerne enfin un extrait d'algue rouge comprenant, en poids par rapport au poids total de la matière sèche :

- Floridoside 25% - 50%, avantageusement 35% - 50%
- Acide iséthionique 10% - 25%

Dans l'extrait d'algue rouge selon l'invention, le rapport massique floridoside/acide iséthionique est avantageusement compris entre 1 et 5, plus avantageusement entre 1,5 et 4, encore plus avantageusement entre 2 et 3.

L'extrait d'algue rouge est concentré en ces deux osmolytes et les quantités de sels monovalents et de pigments ont été minimisées. En effet, ce deux derniers types de molécules peuvent être gênantes en cosmétique : les sels déstabilisent les formules et les pigments colorent les émulsions. L'extrait d'algue rouge comprend avantageusement uniquement 0,01% à 0,5%, en poids par rapport au poids total de la matière sèche, de sels monovalents (chlorure de sodium, chlorure de potassium) et avantageusement aucun pigment.

Dans le cadre de la présente invention, l'algue rouge appartient avantageusement à la famille des *Gigartinaceae,* plus avantageusement au genre *Mastocarpus,* encore plus avantageusement à l'espèce *Mastocarpus stellatus.* Lorsque l'algue rouge utilisée appartient à l'espèce *Mastocarpus stellatus,* l'extrait ne comprend pas d'isofloridoside dans les limites actuelles de détection utilisées.

L'extrait selon l'invention présente une activité anti-déshydratante et une activité protectrice des kératinocytes (protection contre la déshydratation et protection contre les agressions). L'extrait selon l'invention permet d'augmenter significativement la capacité de rétention d'eau du stratum corneum.

L'association des deux osmolytes purs est plus active que la somme de chacun des osmolytes purs. L'association des osmolytes présente donc un effet synergique, avantageusement sur l'activité anti-déshydratante. L'extrait selon l'invention est encore plus actif que l'association des deux osmolytes purs.

L'extrait d'algue rouge peut avantageusement être obtenu par un procédé d'extraction comprenant une étape d'ultrafiltration à 3 500 - 50 000 Daltons suivie d'une étape de nanofiltration à 100 - 200 Daltons (Da).

Le procédé d'ultrafiltration et de nanofiltration permet notamment l'élimination des insolubles, des grosses molécules et des sels. Ce procédé d'extraction et de purification permet donc de concentrer dans l'extrait obtenu les deux osmolytes (floridoside et acide iséthionique).L'étape de nanofiltration permet notamment l'élimination des sels monovalents, qui dans l'extrait brut représentent une part importante de la matière sèche. Les sels sont toxiques sur les cultures de cellules in vitro. De plus, les sels sont, d'une manière générale, desséchants sur la peau. L'étape d'ultrafiltration est avantageusement effectuée avec une membrane ayant un seuil de coupure compris entre 3 500 et 50 000 Da, plus avantageusement entre 10 000 et 50 000 Da, encore plus avantageusement entre 10 000 et 20 000 Da. L'étape de nanofiltration est avantageusement effectuée avec une membrane ayant un seuil de coupure compris entre 100 et 300 Da, plus avantageusement entre 100 et 200.

L'extrait d'algue rouge peut être avantageusement obtenu par un procédé d'extraction et de purification comprenant les étapes suivantes :

- Percolation d'eau déminéralisée à travers un lit d'algue réhydratée
- Ultrafiltration à 10 000 Da
- Nanofiltration à 100-200 Da
- Décoloration et désodorisation
- Conditionnement.

L'extrait d'algue rouge obtenu est un extrait aqueux, enrichi en les deux osmolytes désirés (floridoside et acide iséthionique).

Le cas échéant, l'extrait d'algue rouge obtenu suite à l'étape de nanofiltration peut être décoloré et désodorisé, par n'importe quelle méthode connue de l'homme du métier, notamment par passage sur charbon actif. Dans le cas d'une décoloration et désodorisation par passage sur charbon actif, on ajoute avantageusement 10% en poids de charbon actif par rapport au poids de la matière sèche. L'extrait d'algue rouge est ensuite conditionné dans un conditionnement stérile. Avantageusement, des conservateurs sont ajoutés à l'extrait d'algue rouge.

Le procédé selon l'invention permet d'obtenir un extrait d'algue rouge dans lequel le ratio floridoside : acide iséthionique est standardisé, quelque soit le ratio initial dans l'algue d'origine (ce ratio dans l'algue rouge à l'état naturel peut fluctuer en fonction des saisons et des conditions extérieures).

Les exemples suivants illustrent la présente invention sans en limiter la portée.

<u>EXEMPLE 1 :</u> Procédé d'extraction

Dans cet exemple, les pourcentages sont indiqués en poids de matière sèche de l'extrait considéré par rapport au poids de la matière sèche de l'extrait de départ.

Dans un premier temps, on effectue une extraction aqueuse par de l'eau déminéralisée, pendant 1 heure à température ambiante, de 15 kg de *Mastocarpus stellatus* sec et de 300 kg d'eau. On effectue ensuite une séparation solide/liquide par essorage. On récupère 269 kg d'extrait à 1,3% de matière sèche (MS). Le rendement massique de cette étape d'extraction est de 25% en MS.

Dans une seconde étape, on effectue une ultrafiltration de l'extrait ainsi obtenu sur des membranes ayant un seuil de coupure de 50 000 Da. On récupère le rétentat : 90 kg à 0,5% de MS et le perméat est de 269 kg à 1% de MS. Le rendement massique global, de cette seconde étape, est de 83% en MS.

Dans une troisième étape, on effectue une nanofiltration de l'ultrafiltrat (rétentat obtenu suite à l'étape précédente) sur des membranes ayant un seuil de coupure de 100 à 300 Da. On récupère le rétentat : 91 kg à 1,3% de MS et le perméat est de 480 kg à 0,2% de MS. Le rendement massique global, de cette troisième étape, est de 28% en MS. Le nanofiltrat (rétentat obtenu suite à l'étape précédente) est ensuite décoloré sur cartouche de charbon actif CECA ENO. On récupère 86 kg d'extrait d'algue rouge à 1,1% de MS. Le rendement massique de cette étape est de 79% en MS.

Le rendement global massique des étapes d'extraction, de purification et de décoloration est de 7% en MS.

EXEMPLE 2 : compositions selon l'invention

Association :
25% extrait algue rouge
25% peptides de spiruline
13% eau de mer
37% eau osmosée

| CREME DE JOUR | % |
|---|---|
| Huiles végétales | 4 |
| Emulsionnants | 5 |
| Esters | 9 |
| Emollient | 2 |
| Filtres solaires | 6 |
| Antioxydant | 0.5 |
| Ecrans minéraux | 4 |
| Eau osmosée | qsp100 |
| Agent chélatant | 0,1 |
| Gélifiant | 1,6 |
| Humectant | 2 |
| Agent de toucher | 1 |
| Silicone | 6 |
| Conservateurs | 0,6 |
| Actif anti-age | 3 |
| Parfum | 0,5 |
| **Actif association** | **5** |

| CREME DE NUIT | % |
|---|---|
| Huiles végétales | 7 |
| Emulsionnants | 5 |
| Co-emulsionnant | 2 |
| Esters | 5 |
| Emollient | 6 |
| Antioxydant | 0,5 |
| Eau osmosée | qsp100 |
| Agent chélatant | 0.1 |
| Gélifiant | 1 |
| Humectant | 2 |
| Silicone | 5 |
| Conservateurs | 0,7 |
| Actif anti-age | 3 |
| Actif marin | 1 |
| Parfum | 0,25 |
| Colorant | qs |
| Ajusteur de pH | qs |
| **Actif association** | **5** |

(suite)

| | |
|---|---|
| CREME DE NUIT | % |
| **Peptides de spiruline** | **1,25** |
| LAIT DEMAQUILLANT | % |
| Eau osmosée | qsp100 |
| Humectant | 3 |
| Gélifiant | 0,3 |
| Esters | 8 |
| Ajusteur de Ph | qs |
| Conservateurs | 0,3 |
| Parfum | 0,2 |
| Colorant | qs |
| **Actif association** | **1** |
| MASQUE | % |
| Eau osmosée | qsp100 |
| Humectant | 10 |
| Gélifiant | 1 |
| Huiles végétales | 13 |
| Emollient | 3 |
| Conservateurs | 0,3 |
| Actif « relaxant » | 3 |
| Parfum | 0,2 |
| **Actif association** | **5** |

qs = quantité suffisante
qsp100 = quantité suffisante pour atteindre 100%

EXEMPLE 3 : Mesure de la rétention d'eau : activité anti-déshydratante

- *Composition du produit :*

On utilise un extrait de *Mastocarpus stellatus à* 10% en poids de matière sèche, par rapport au poids total de l'extrait, présentant une teneur en osmolytes, en poids par rapport au poids total de la matière sèche de l'extrait :

Floridoside : 38%
Acide iséthionique : 19%

Dans la suite de l'exemple, cet extrait est dénommé « extrait de *mastocarpus* 10% MS».

- *Protocole Technique :*

On étudie l'hydratation du stratum corneum (SC) délipidisé et traité par différentes solutions aqueuses.

Le but de cette étude est de montrer si l'application cutanée d'une solution aqueuse d'extrait de mastocarpus (10% de matière sèche), de floridoside pur à 3,8%, d'acide iséthionique pur à 1,9%, d'un mélange de floridoside pur à 3,8% et d'acide iséthionique pur à 1,9%, modifie significativement l'état d'hydratation du SC délipidisé puis conservé dans une enceinte à 25°C et à 40 % d'humidité relative (RH).

Dans cette étude, des échantillons de SC, isolés de l'épiderme de morceaux d'oreilles de porc par un traitement à la trypsine (1% dans un tampon phosphate; pH=7,4) puis délipidisés par un solvant (acétone par exemple), ont été immergés pendant 8 heures dans une solution aqueuse d'extrait de mastocarpus 10%, de floridoside pur à 3,8%, d'acide iséthionique pur à 1,9%, d'un mélange de floridoside pur à 3,8% et d'acide iséthionique pur à 1,9%, (3 échantillons de SC par produits). Trois échantillons de SC délipidisé ont été traités par de l'eau ultra pure (contrôle, 18 MΩ), pendant 8 heures. Après traitement, ces échantillons ont été pesés puis placés dans une étuve maintenue à 25°C et à 40% RH. La masse des échantillons de SC traités par chacune des solutions a été déterminée après 1h; 2h; 3h; 4h; 5h et 6h de conservation à 25°C et à 40% RH.

Afm de déterminer la réhydratation des échantillons de SC, l'humidité relative a été augmentée de 40% à 60% entre 6h et 24h (25°C). Après 24h, la rétention d'eau dans le SC a été calculée à partir de l'équation 1 (n=24h).

La rétention d'eau dans le SC (water-holding capacity, WHC) a été calculée à partir de la variation de masse du SC, comme suit :

$$\text{WHC}(\%) = [1 - ((\text{masse SCo} - \text{masse SCn}) / \text{masse SCO})] \times 100 \ (\textit{Equation 1})$$

masse SC0 : masse du SC à t=0
masse SCn : masse du SC à t = n
n = 1h ; 2h ; 3h ; 4h ; 5h ; 6h et 24h.

- *Résultats et discussion*

Les résultats sont reportés sur la figure 1, qui représente la variation relative de la capacité de rétention (WHC) du SC traité par chacune des solutions décrites ci dessus. Chaque valeur est la moyenne ± l'écart type de 3 déterminations expérimentales.

La Figure 1 montre les variations relatives de la WHC du SC traité pendant 8 heures par une solution aqueuse (i) d'extrait de *Mastocarpus,* de floridoside pur à 3,8%, d'acide iséthionique pur à 1,9%, d'un mélange de floridoside pur à 3,8% et d'acide iséthionique pur à 1,9%.

Légende :

eau ultrapure témoin
*mastocarpus* 10% (matière sèche)
acide iséthionique 1,9%(matière sèche)
floridoside 3,8%(matière sèche)
acide iséthionique 1,9% + floridoside 3,8%(matière sèche)

Après 6 heures de conservation à 25°C et 40% RH, les échantillons de SC traités par l'extrait de *Mastocarpus* sont significativement moins déshydratés que les échantillons contrôles. De même, les échantillons de SC traités par la solution aqueuse de floridoside à 3,8% et d'acide iséthionique à 1,9% d'une part, ou par la solution aqueuse d'acide iséthionique à 1,9% sont moins déshydratés que les échantillons contrôles. L'état de déshydratation des échantillons de SC traité par une solution aqueuse de floridoside à 3,8% et par l'eau n'était pas significativement différent.

- *Conclusion :*

L'histogramme présenté sur la figure 2 représente le pourcentage de rétention d'eau qui est calculé par l'opération suivante : la différence entre le pourcentage WHC des extraits ou des molécules et le pourcentage WHC du témoin. Sur cette figure 2 une série a été rajoutée, elle correspond à la somme de la série « floridoside 3,8% » et de la série « acide iséthionique 1,9% ».

Légende :

*mastocarpus* 10% (matière sèche)
acide iséthionique 1,9% (matière sèche)
floridoside 3,8% (matière sèche)
acide iséthionique 1,9% + floridoside 3,8% (matière sèche)
somme floridoside 3,8% et acide iséthionique 1,9% (matière sèche)

L'extrait de *Mastocarpus* augmente entre 24% et 29% la rétention d'eau du stratum corneum et présente une activité supérieure aux osmolytes purs testés individuellement ou en mélange. L'extrait de *Mastocarpus* est plus actif que ces osmolytes purs. L'association des deux osmolytes présente un effet synergique sur la rétention d'eau. En effet le mélange des deux molécules présente une activité supérieure (entre 2% et 8% selon le temps d'exposition) à la somme des activités des deux osmolytes testés individuellement.
L'extrait de *Mastocarpus* présente donc un grand intérêt dans la formulation de produits cosmétiques à activité anti-déshydratante.

EXEMPLE 4 : Evaluation de l'effet protecteur sur des kératinocytes humains normaux exposés à un choc thermique

Cette étude a consisté à tester l'effet protecteur de l'extrait de *Mastocarpus stellatus,* sur des kératinocytes humains normaux exposés à un choc thermique ainsi qu'à étudier l'effet modulateur de ces extraits sur l'expression de la protéine HSP70 («Heat-Shock proteins», protéines de choc).

- *Composition du produit :*

L'extrait de *Mastocarpus* est sous forme lyophilisée (100% en poids de matière sèche) et dilué aux concentrations désirées. La teneur en osmolytes, en poids par rapport au poids total de la matière sèche, dans l'extrait est de : Floridoside 38% et acide iséthionique 19%.

- *Protocole:*

Cette étude a été réalisée sur des kératinocytes humains normaux cultivés en monocouche dans du milieu de culture KGM (keratinocyte growth medium : milieu de croissance de kératinocytes) supplémenté, à 37°C, en atmosphère humide et en présence de 5 % de $CO_2$. Lors du deuxième passage, les kératinocytes ont été ensemencés dans des plaques 24 puits pour les tests de viabilité cellulaire et dans des labtecks pour la réalisation des études immunocytochimiques.

A) Evaluation de la viabilité des kératinocytes :

A 50 % de la confluence, les kératinocytes ont été incubés en présence de l'extrait de mastocarpus à différentes concentrations. La viabilité des kératinocytes a alors été évaluée par un test MTT (méthyle thiazolyl tétrazolium) à 24 h et 48 h et comparée à celle du milieu contrôle (milieu de culture seul).
Le MTT est en effet converti par les déshydrogénases cellulaires en un dérivé coloré insoluble du formazan qui est alors solubilisé dans l'isopropanol acidifié. Ce test colorimétrique permet ainsi de lier l'intensité de la coloration au nombre de cellules vivantes. Les doses choisies sont inférieures aux doses cytotoxiques.

- *Résultats :*

L'extrait de *Mastocarpus* s'est avéré toxique sur les kératinocytes cultivés en monocouche lorsqu'ils sont utilisés à des concentrations supérieures ou égales à 0,05 % (poids/volume de matière active) pour l'extrait de *Mastocarpus* à t=24 h ou à des concentrations supérieures ou égales à 0,55 % (poids/volume de matière active) à t=48h.

B) Evaluation de l'effet protecteur de l'extrait de *Mastocarpus* sur la viabilité kératinocytaire vis à vis d'un choc thermique

Les kératinocytes à confluence depuis 2 jours (afin d'éviter les effets liés au cycle cellulaire) ont été incubés en présence de l'extrait à tester (à 3 concentrations différentes : 0,025%, 0,04% et 0,05% en poids de matière sèche) pendant 24 h. Le milieu de culture seul a servi de milieu contrôle. Les kératinocytes ont alors été exposés pendant 90 minutes à 42°C (ou à 37°C pour le contrôle) puis ont été remis à 37°C.
La viabilité des kératinocytes a alors été évaluée 22,5 heures plus tard par un test MTT et comparée à celle des kératinocytes du milieu contrôle.
La viabilité des kératinocytes exposés pendant 90 minutes à 42°C est diminuée de 23 % (+/- 16,4, p=0,002) par rapport à celle des kératinocytes incubés à 37°C.
A 42°C, la viabilité des kératinocytes incubés en présence des différents extraits est supérieure à celle des kératinocytes incubés avec le milieu de culture seul. La viabilité cellulaire est en effet augmentée, par rapport au milieu de culture seul (milieu contrôle), tel que cela ressort du tableau 1 suivant :

Tableau 1 : viabilité cellulaire à 42°C en présence d'un extrait de mastocarpus

| | % modulation | Ecart-type (Test Student) |
|---|---|---|
| Extrait *mastocarpus* 0,025 % MS | + 15 % | ± 12,4 (p=0,01) |
| Extrait mastocarpus 0,04 % MS | + 26,2 % | ±14,8 (p=0,01) |
| Extrait *mastocarpus* 0,05 % MS | + 28,4 % | ±14,8 (p=0,01) |
| MS = matière sèche | | |

C) Evaluation de l'effet modulateur de l'extrait de *Mastocarpus* sur l'expression de la protéine HSP70 :

Les kératinocytes sont des cellules qui en cas de stress vont synthétiser ces HSPs.

A 50 % de la confluence, les kératinocytes ont été incubés, pendant 24 heures, en présence de l'extrait de *Mastocarpus* à 3 concentrations différentes : 0,025%- 0,04% et 0,05% de matière sèche. Le milieu de culture seul a servi de milieu contrôle. Les l'expression de la protéine HSP70, dont la synthèse est stimulée par un choc thermique, a alors été étudiée par une technique d'immunocytochimie.

Les résultats sont montrés sur la figure 3, qui représente le pourcentage d'amélioration de la viabilité cellulaire par l'extrait de *Mastocarpus* par rapport au témoin sur des kératinocytes incubés. La figure 4 représente l'expression de la protéine HSP70 par les kératinocytes humains normaux en culture in vitro et incubés ou non avec l'extrait de *Mastocarpus* selon l'invention.

Légende :

cellules mortes

faible expression

expression modérée

forte expression

L'extrait de *Mastocarpus* (aux trois concentrations) protège de manière significative les kératinocytes de la mortalité cellulaire. Il augmente également le niveau d'expression de la protéine HSP-70 dans les kératinocytes incubés à 42°C, et notamment le pourcentage de kératinocytes exprimant la protéine HSP-70. L'extrait de *Mastocarpus,* augmente également l'intensité d'expression de la protéine HSP-70 par les kératinocytes humains normaux en culture in vitro.

- *Conclusion*

L'extrait de *Mastocarpus* n'exerce pas d'effet modulateur sur la prolifération et la différenciation kératinocytaire. Il protège les kératinocytes de la mort cellulaire lors d'une exposition à une température de 42°C via une modulation de l'expression de la protéine HSP-70.

CONCLUSION GENERALE SUR L'EXTRAIT D'ALGUE ROUGE : L'extrait de *Mastocarpus stellatus* riche en floridoside et en acide iséthionique présente des activités de protection de la peau et des cellules de la peau qui se manifestent par une activité anti-déshydratante et par une activité protectrice sur les kératinocytes dont la synthèse des protéines de stress HSP 70 (Heat Shock Protein 70) est induite et stimulée.

EXEMPLE 5 : Etude de l'association extrait d'algue rouge + extrait peptidique de spiruline + eau de mer

Dans cet exemple, l'association d'extrait d'algue rouge 25% *(Mastocarpus Stellatus),* d'extrait peptidique de spiruline 25% et d'eau de mer 13%, eau osmosée 37% a été testée sur différents modèles cellulaires. Les pourcentages sont exprimés en poids par rapport au poids total de l'association.

matière sèche de l'eau de mer : 3% p/p
matière sèche de l'extrait d'algue rouge : 1% p/p
matière sèche de l'extrait peptidique de spiruline : 5% p/p

Cette association est dans la suite dénommée « association testée » ou «association ».

I Effet sur l'induction de HSP70 par la chaleur dans des cultures de kératinocytes :

Cette étude a permis d'évaluer les effets de l'association des actifs (extrait peptidique de spiruline, extrait de mastocarpus et eau de mer) sur la quantité de HSP70 dans des kératinocytes humains exposés ou non à la chaleur. La mesure a été réalisée en utilisant un anticorps fluorescent spécifique de la protéine HSP70 et par mesure à l'aide de la méthode de cytométrie de flux.

- Matériels & Méthodes
- *Modèles biologiques*

Type : kératinocytes épidermiques humains normaux (NHEK)
Culture : 37°C, 5 % CO2
Milieu: milieu SFM (Serum Free Medium = milieu sans sérum) EGF (epidermal growth factor = facteur de croissance épidermal) 0,25 ng/ml et extrait pituitaire (EP) 25 ng/ml Gentamycine 25 ug/ml

*- Produit à l'essai*

On a testé ladite association à la concentration de 0,22% en poids de matière sèche, par rapport au poids total (dilution en milieu de culture). A cette concentration, le produit n'est pas cytotoxique.

*- Traitements et analyse*

Les cellules (NHEK) ont été ensemencées. A confluence, les cellules ont été traitées ou non (témoin) par l'association et incubées pendant 2 heures à 37°C. A la fm du traitement, une des séries de plaques a été placée à 45°C pendant 15 minutes (cellules exposées à la chaleur). Puis le milieu de culture de toutes les plaques a été éliminé et remplacé par du milieu neuf contenant ou non le produit. Les cellules ont ensuite été cultivées pendant 6 h à 37°C et 5 % de CO2.
Après incubation, les surnageants de culture ont été éliminés et les tapis cellulaires ont été rincés avec une solution de PBS (phosphate-buffered saline = solution saline tamponnée phosphatée) puis les cellules ont été trypsinisées. Les cellules ont été fixées par traitement TBS (Tris-Buffered Saline = Solution saline tris-tamponnée)/Paraformaldéhyde pendant une nuit à 4°C et à l'obscurité puis lavées en PBS.
Après perméabilisation, le marquage a été réalisé par un anticorps primaire spécifique du marqueur HSP70 couplé à la FITC (Anti-Hsp70).
Après plusieurs lavages en PBS, la mesure des paramètres de fluorescence du marqueur HSP-70 a été évaluée par cytométrie de flux sur 10 000 cellules individuelles par échantillon à l'aide d'un cytomètre.

- Résultats & Conclusion
- *Effets sur la quantité relative de HSP-70*

Le signal basal de fluorescence obtenu dans le contrôle sans anticorps anti-HSP70 était négligeable (intensité de fluorescence de 9 mais seulement 10 % de la population ayant répondu ; 10 % de cellules positives).

*Kératinocytes non exposés à la chaleur*

Tableau 2 Quantité relative de HSP70 - Cellules non exposées à la chaleur

| Traitement | Intensité de fluorescence (n =10 000 cellules) - moyenne | Ecart type | Nombre d'échantillons | % témoin | Probabilité (test de student) |
|---|---|---|---|---|---|
| Témoin | 11,59 | 0,27 | 3 | 100 | - |

(suite)

| Traitement | Intensité de fluorescence (n =10 000 cellules) - moyenne | Ecart type | Nombre d'échantillons | % témoin | Probabilité (test de student) |
|---|---|---|---|---|---|
| Association 0,22% de MS | 11,39 | 0,22 | 3 | 98 | p>0,05 |
| Détermination basée sur la mesure de fluorescence de l'anticorps anti-HSP70-FITC (n =10000 cellules analysées). | | | | | |

Chaque valeur est la moyenne ± l'écart type de 3 déterminations expérimentales. Dans ces conditions expérimentales, la quantité relative de HSP-70 produite par les kératinocytes non exposés à la chaleur était faible mais détectable (intensité de fluorescence de 11 seulement pour 70 % de cellules positives).

L'association testée (extrait d'algue rouge, extrait peptidique de spiruline et eau de mer) n'a pas modifié la quantité relative de HSP-70.

*Kératinocytes exposés 15 min à 45°C*

Tableau 3 Quantité relative de HSP70 • Cellules exposées 15 min à 45°C

| Traitement | Intensité de fluorescence (n = 10000 cellules) - moyenne | Ecart type | Nombre d'échantillons | % témoin | Probabilité (test de student) |
|---|---|---|---|---|---|
| Témoin | 74,05 | 4,09 | 3 | 100 | - |
| Association 0,22% MS | 96,07 | 7,42 | 3 | 130 | P < 0,05 |
| Détermination basée sur la mesure de fluorescence de l'anticorps anti-HSP70-FITC (n =10000 cellules analysées). | | | | | |

Chaque valeur est la moyenne ± l'écart type de 3 déterminations expérimentales. Le traitement des kératinocytes par la chaleur a significativement augmenté la quantité relative de HSP-70 (stimulation d'un facteur 6 environ). Ce résultat attendu valide l'essai. Dans ces conditions expérimentales, l'association testée (extrait d'algue rouge, extrait peptidique de spiruline et eau de mer) à 0,22% de matière sèche a permis d'augmenter la quantité relative de HSP70 induite par la chaleur (130 % du témoin, p<0,05, soit une amélioration de 30%).

- *Conclusion :*

L'association testée (extrait d'algue rouge, extrait peptidique de spiruline et eau de mer) à 0,22% de matière sèche augmente significativement (+30%) la synthèse des HSPs70 en cas de stress thermique.

II Effet sur la croissance et la différenciation de Kératinocytes humains

Cette étude a permis d'évaluer les effets de l'association (extrait d'algue rouge, extrait peptidique de spiruline et eau de mer) sur la différentiation par mesure de l'activité transglutaminase kératinocytaire (TGk) dans des cultures de kératinocytes et sur la prolifération par mesure de l'incorporation de thymidine par des kératinocytes.

- *Modèles biologiques*

Type : kératinocytes épidermiques humains normaux (NHEK)
Culture : 37°C, 5 % $CO_2$
Milieu pour TGK: milieu SFM EGF 0,25 ng/ml et extrait pituitaire (EP) 25 ng/ml Gentamycine 25 ug/ml
Milieu pour prolifération : milieu SFM Sans EGF et EP Gentamycine 25µg/ml

- *Produit à l'essai*

On a testé ladite association à 2 concentrations : 0,22% et 0,11% en poids de matière sèche, par rapport au poids total (dilution en milieu de culture). A ces concentrations, les produits sont non cytotoxiques.

Rétinol : concentration testée : $10^{-6}$ moles (dilution en milieu de culture)
Acide rétinoïque : concentration testée : $10^{-7}$ moles (dilution en milieu de culture)
Chlorure de calcium : concentration testée : 1,5 mM (dilution en milieu de culture stérile)

- *Activité transglutaminase (TGk) kératinocytaire*

Les kératinocytes ont été ensemencés (10000 cellules/puits) et pré-cultivés en milieu SFM. Après incubation pendant 24 heures à 37°C, le milieu de culture a été éliminé et remplacé par du milieu SFM contenant ou non (témoin) l'association ou les références rétinol, acide rétinoïque et chlorure de calcium. Après 48h d'incubation à 37°C et 5 % de $CO_2$, le milieu de culture a été éliminé et les cellules ont été à nouveau traitées puis incubées pendant 48h à 37°C. Les tapis cellulaires ont été lavés puis soniqués, sur glace, en tampon Tris/EDTA (Acide éthylène diamine tétraacétique), pH 8,0. L'enzyme TGk membranaire a été extraite, puis l'activité TGk a été déterminée.

- *Incorporation de thymidine par les NHEK*

- Marquage métabolique

Les kératinocytes ont été ensemencés à 8000 cellules/puits, en plaque 96 puits et en milieu SFM complet. Après 24 h d'incubation à 37°C et 5 % de $CO_2$ le milieu de culture a été éliminé et remplacé par du milieu SFM -EP, -EGF puis les cellules ont été cultivées pendant 24h. A 30 % de confluence, le milieu a été éliminé et remplacé, par du milieu SFM -EP, -EGF contenant ou non (témoin) l'association ou par du milieu +Ep, +EGF (référence). Les cellules ont ensuite été cultivées pendant 48h à 37°C et 5 % de CO2 avec addition de 10 μCi/ml fmal de [méthyl-3H]-thymidine pendant les 24 dernières heures.

- Analyse de la radioactivité incorporée dans les puits de culture, ajout de 1 V de tampon chaotropique*
- précipitation acide trichloracétique (TCA) collection sur filtres
- lavages TCA puis éthanol 70 % comptage en scintillation liquide

*Résultats*

- *Activité transglutaminase (TGk) kératinocytaire*

Tableau 4 : Activité de la transglutaminase sans calcium dans le milieu

| Traitement | | Activité TGk (cpm) | Ecart type | Nombre d'échantillons | % témoin | Probabilité (test de Student) |
|---|---|---|---|---|---|---|
| Témoin | - | 865 | 124 | 12 | 100 | - |
| Chlorure de calcium | 1,5mM | 3408 | 660 | 12 | 394 | P < 0,01 |
| Rétinol | $10^{-6}$M | 465 | 109 | 6 | 54 | P < 0,05 |
| Acide rétinoïque | $10^{-7}$M | 447 | 74 | 6 | 52 | P < 0,05 |
| Association testée | 0,22% MS | 3308 | 468 | 6 | 383 | P < 0,01 |
| | 0,11% MS | 1809 | 282 | 6 | 209 | P < 0,01 |

Tableau 5 : Activité de la transglutaminase par rapport à la quantité de protéines.

| Traitement | | Activité TGK (cpm) | µg prot | activité/µg | % |
|---|---|---|---|---|---|
| Témoin | - | 865 | 4,38 | 197 | 100 |
| Chlorure de calcium | 1,5 mM | 3408 | 5,44 | 626 | 317 |
| Rétinol | 10-6 M | 465 | 3,27 | 142 | 72 |
| Acide retinoïque | | 447 | 3,41 | 131 | 67 |
| Association testée | 0,22% MS<br>0,11% MS | 3308<br>1809 | 6,23<br>5,81 | 531<br>312 | 269<br>158 |

Les résultats du dosage ont montré une activité transglutaminase de l'ordre de 4 fois plus forte en milieu calcium (inducteur de la différenciation des NHEK) qu'en milieu sans calcium. Comme attendu, les rétinoïdes de référence ont réduit de façon significative l'activité TGk des cultures. L'acide rétinoïque testé à $10^{-7}$ M a provoqué une réduction de l'activité TGk de 46 %. Le rétinol testé à $10^{-6}$ M a provoqué une réduction de l'activité TGk de 48 %. Ces résultats étaient attendus et valident l'essai.

Dans les conditions expérimentales de cet essai, l'association testée à 0,22% et 0,11% de matière sèche a provoqué une augmentation significative de l'activité TGk présente dans les cultures de NHEK (respectivement 383 % et 209 % par rapport au témoin non traité, p<0,01). Malgré une augmentation de la quantité de protéines, cet effet restait significatif lorsque l'activité TGk a été rapportée à la quantité de protéines (269 % et 158 %, voir tableau 5). L'augmentation de la quantité de protéines est logique puisque lors de la différenciation, les kératinocytes synthétisent beaucoup de protéines de structure telles les kératines et la filaggrine.

En conclusion, l'association testée à 0,22% et 0,11% de matière sèche a présenté une activité pro-différenciante dans les conditions expérimentales de cet essai (paramètre TGk).

- *Prolifération des kératinocytes*

Tableau 6 : Prolifération des NHEK (lignée de kératinocytes)- Incorporation de Thymidine

| Traitement | | Acitivté TGK (cpm) | Ecart type | Nombre d'échantillons | % témoin | Probabilité (test de student) |
|---|---|---|---|---|---|---|
| Témoin | - | 5015 | 629 | 3 | 100 | - |
| Contrôle<br>EGF + EP | EGF 0,25 ng/ml<br>EP 25 µq/ml | 8053 | 847 | 3 | 161 | P < 0,01 |
| Association testée | 0,22% MS | 2625 | 123 | 3 | 52 | P < 0,01 |
| | 0,11 % MS | 3695 | 273 | 3 | 74 | P < 0,05 |

Le mélange « EGF + extrait pituitaire » a stimulé significativement l'incorporation de thymidine par les kératinocytes (facteur > 1,5 par rapport au témoin, p<0,01). Ce résultat valide l'essai.

Dans les conditions expérimentales de cet essai, l'association testée à 0,22% et 0,11 % de matière sèche a diminué significativement l'incorporation de thymidine par les NHEK (respectivement 52 % et 74 % du témoin non traité, p<0,01 et 0,05).

En conclusion, l'association testée à 0,22% et 0,11% de matière sèche a diminué la prolifération des kératinocytes.

*Conclusion*

Dans cet essai in vitro, l'association (extrait d'algue rouge, extrait peptidique de spiruline et eau de mer) a montré un effet dose-dépendant de stimulation de la différenciation des kératinocytes. Cet effet était logiquement corrélé à un effet anti-prolifération sur ces cellules.

III Effet sur les fibroblastes :

- *Obtention et amplification des fibroblastes*

Des prélèvements cutanés (punch-biopsie de 3 mm de diamètre) sont réalisés sur une abdominoplastie provenant d'une femme âgée de 40 ans. La technique d'explantation classique est utilisée pour l'extraction des fibroblastes.

- *Préparation des échantillons*

L'association sous forme lyophylisée est diluée dans du DMEM (Dulbecco's Modified Eagle Medium = mélange milieu de Eagle modifié par Dulbecco) et amenée à la concentration de 0,11 % en matière sèche.

- *Dosage de la synthèse de collagène*

a) Méthode

Une méthode colorimétrique est utilisée pour doser la synthèse de collagène. Le réactif coloré est un réactif contenant des groupements acide sulfonique qui se fixent sur les groupements aminoacides présents dans le collagène. Le complexe formé est dissocié par un réactif alcalin. La coloration développée est mesurée grâce à un spectrophotomètre.
Les fibroblastes sont cultivés en présence de l'association (0,11% de matière sèche) pendant 1, 2, 3 et 4 semaines. La gamme étalon (0-50 μg) est réalisée à partir d'une solution standard de collagène.
Les échantillons à tester sont dilués au 1/2. Les échantillons et la gamme étalon sont incubés pendant 30 minutes à température ambiante sous agitation avec 1 ml de réactif coloré. Ils sont ensuite centrifugés 10 mn à 10 000 g. Les surnageants sont éliminés et les culots sont dissous dans 1 ml de réactif alcalin de dissociation. Une coloration se développe en 10 mn. L'absorbance de chaque échantillon est mesurée à 540 nm. Les résultats sont exprimés en μg de collagène par mg de protéines totales.

b) résultats

Les résultats sont donnés sur la figure 5.

Légende :

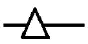 Témoin

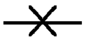 Association testée (0,11% de matière sèche) versus témoin

L'association testée, utilisée au pourcentage 0,11% MS, stimule significativement la synthèse de collagène, de 44% à 4 semaines de culture.

- *Dosage de l'élastine*

a) Méthode

Une méthode colorimétrique est utilisée pour dose l'élastine totale soluble :

- le réactif est le 5,10,15,20-tétraphényl-21,23-porphine sulfonate (TTPS) qui se fixe spécifiquement sur l'élastine
- Le réactif de précipitation contient de l'acide trichloroacétique et de l'arginine
- Le complexe formé est dissocié par une solution de guanidine-HCl et de propane-1-ol
- La coloration développée est mesurée grâce à un spectrophotomètre

Les fibroblastes sont cultivés en présence de l'association testée à 0,11% en poids de matière sèche pendant 1, 2, 3 et 4 semaines.
Une gamme étalon (0-75 μg) a été réalisée à partir d'une solution standard d'élastine.
Une prise d'essai de 100 μl est effectuée à partir des échantillons à tester (les échantillons ne sont pas dilués).

Les échantillons et la gamme étalon sont incubés, après agitation, pendant une nuit dans de la glace au réfrigérateur avec 1ml de réactif de précipitation. Ils sont ensuite centrifugés à température ambiante pendant 10 mn à 10 000g. Les surnageants sont éliminés et les culots sont dissous dans 1 ml de TPPS puis incubés pendant une heure à température ambiante sous agitation. Ils sont ensuite centrifugés 10 mn à 10 000g. Les surnageants sont éliminés et les culots sont dissous dans 1 ml de réactif de dissociation. Une coloration se développe et l'absorbance de chaque échantillon est mesurée à 513 nm avec un spectrophotomètre. Les résultats sont exprimés en µg d'élastine par mg de protéines totales.

b) Résultats

Les résultats sont donnés sur la figure 6.

Légende :

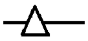 Témoin

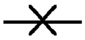 Association testée (0,11% de matière sèche) versus témoin

L'association testée à 0,11% MS augmente significativement la synthèse d'élastine de 43% à 4 semaines de culture.

- *Conclusion*

L'association testée (extrait d'algue rouge, extrait peptidique de spiruline et eau de mer), à la dose 0,11 % MS, stimule la synthèse de certains composants de la matrice extracellulaire dont le collagène et 1' élastine (respectivement de +44% et +43%).

IV Effet Botox like (effet anti-ride)

Cette étude a été réalisée dans le but d'analyser les effets de l'association testée sur la modulation de la fréquence de contraction de fibres musculaires en coculture avec des explants de moelle épinière.

Matériels et Méthodes

- *Cellules utilisées*

Type : Primo culture de cellules musculaires humaines (Mulc)

Explants de moelle épinière avec ganglions rachidiens prélevés sur des embryons de rat Wistar de 13 jours de gestation.

Milieu de culture : Mélange de 2/3 de MEM et de 1/3 de M199
L-glutamine 2mM
Pénicilline 50 UI/ml - Streptomycine 50 ug/ml
Sérum de veau foetal 5 % (v/v)
Culture : 37°C et à 5% de $CO_2$

- *Produits et mélanges à l'essai*

On a testé ladite association à la concentration de 1% en poids de matière sèche, par rapport au poids total (dilution en milieu de culture). A cette concentration, le produit est non cytotoxique.

Carisoprodol : concentration de 10$^{-3}$M (dilution en milieu de culture).

- *Coculture cellules musculaires et explants de moelle épinière.*

Les myoblastes humains ont été ensemencés en puits de culture. A confluence, des explants de moelle épinière d'embryons de rat de 13 jours ont été déposés sur la culture. Dans ces conditions, les premières contractions des fibres musculaires ont été observées après une semaine de coculture. Après 3 semaines, ces fibres musculaires sont striées

et possèdent des jonctions neuromusculaires différenciées matures.

- *Traitement des cultures et analyses de la fréquence de contraction*

Les cultures ont été observées à l'aide d'un microscope inversé équipé d'une caméra pour l'enregistrement de séquence vidéo et d'une platine.

Pour chaque point expérimental, un puits de culture a été utilisé, dans lequel une fibre musculaire ayant des contractions régulières (fréquence supérieure à 60 contractions par minute) a été sélectionnée et le nombre de contractions a été comptabilisé sur 30 secondes. Sans bouger la platine du microscope, le produit a été incubé dans le milieu pendant 60 secondes. A la fin de cette incubation, le nombre de contractions a été déterminé sur 30 secondes. Les cultures ont été replacées dans l'incubateur puis, après 2 heures d'incubation, le nombre de contractions pendant 30 secondes de chacune des fibres sélectionnées, a été déterminé.

Après 48 heures d'incubation en présence des produits, une analyse visuelle fine des cellules en culture a été réalisée pour vérification de la viabilité cellulaire. Afm d'éliminer l'inhibition possible de la fréquence de contraction liée au manque de glucose, du milieu de culture contenant 5 g/l de glucose a été ajouté dans les surnageants de culture (solution finale à 1 g/l de glucose). Vingt secondes après cette recharge en glucose, le nombre de contractions pendant 30 secondes a été déterminé pour chacune des fibres sélectionnées.

Uniquement pour les puits de culture contenant les fibres musculaires bloquées, le milieu de culture a été éliminé et du milieu de culture contrôle sans produit a été ajouté. Les cultures ont été incubées pendant 2 heures supplémentaires. A la fin de cette incubation, le nombre de contractions pendant 30 secondes a été déterminé pour chacune des fibres sélectionnées.

Pour analyser les résultats, les paramètres suivants ont été sélectionnés :

- une diminution de 25 % de la fréquence de contraction n'est pas significative.
- une diminution de la fréquence de contraction comprise entre 25 % et 75 % est considérée comme un ralentissement de la contraction.
- une diminution supérieure à 75 % est considérée comme un blocage de la contraction.

Résultats : Modulation de la fréquence de contraction

- *Témoin milieu*

Après 1 minute et 2 heures d'incubation, le témoin milieu n'a pas modifié la fréquence de contraction des 3 fibres sélectionnées. Après 48 heures d'incubation du témoin milieu et recharge en glucose, toutes les fibres musculaires présentaient une fréquence de contraction proche de leur fréquence de contraction initiale.

- *Carisoprodol*

Le carisoprodol est un produit myorelaxant de référence agissant au niveau de la jonction neuromusculaire. Après 1 minute et 2 heures d'incubation, le carisoprodol à $10^{-3}$ M a bloqué la fréquence de contraction des 3 fibres musculaires sélectionnées. L'apport de glucose à 48 heures n'a pas permis aux fibres musculaires de reprendre une activité contractile. Après changement de milieu et incubation sans produit pendant 2 heures supplémentaires, les fibres musculaires ont récupéré une fréquence de contraction proche de leur fréquence de contraction initiale (Le pourcentage moyen de contraction des trois fibres est 111%).

- *Conclusion :* le carisoprodol, à la concentration de 0,03% a bloqué, la fréquence de contraction des fibres musculaires en culture après 1 minute, 2 heures et 48 heures d'incubation. L'arrêt des contractions était réversible.
  Les résultats obtenus avec le carisoprodol et le témoin milieu ont permis de valider l'expérience après 1 minute, 2 heures et 48 heures d'incubation.

- *Association testée*

Les résultats sont présentés dans la figure 7.

Légende :

témoin

carisoprodol 0,03%

association 1% MS

association 0,22% MS

association 0,11% MS

Après 1 minute et 2 heures d'incubation, l'association testée à 1% de matière sèche a bloqué la contraction des 3 fibres musculaires sélectionnées (aucune contraction). Après 48 heures d'incubation et recharge en glucose, les 3 fibres musculaires étaient toujours bloquées (aucune contraction).

Après changement de milieu et incubation sans produit pendant 2 heures supplémentaires, 2 des 3 fibres musculaires sélectionnées ont récupéré une fréquence de contraction proche de leur fréquence de contraction initiale. La dernière fibre a retrouvé une activité contractile, mais avec une fréquence de contraction ralentie par rapport à sa fréquence de contraction initiale. Le pourcentage moyen de contraction de ces trois fibres est de 90%.

- *Conclusion :* l'association testée à 1% de matière sèche a bloqué la fréquence de contraction des fibres musculaires. L'arrêt de contraction était réversible quand le produit a été retiré des cultures. Aux concentrations inférieures cet actif n'a pas eu d'effet sur l'activité contractile des fibres musculaires.

CONCLUSION GENERALE SUR L'ASSOCIATION EXTRAIT D'ALGUE ROUGE, EXTRAIT PEPTIDIQUE DE SPIRULINE ET EAU DE MER :

L'association selon l'invention, constituée d'extrait d'algue rouge, d'extrait peptidique de spiruline et d'eau de mer, présente les propriétés suivantes :

- Amélioration de la différentiation des kératinocytes : activité restructurante de l'épiderme.
- Augmentation de la synthèse des HSP 70 en cas de stress thermique : activité anti-stress
- Inhibition réversible des contractions de fibres musculaires : activité Botox Like.
- Augmentation de la synthèse du collagène et de l'élastine : activité régénérante du derme.

## Revendications

**1.** Composition cosmétique comprenant en tant que principe actif un extrait d'algue rouge comprenant une association de floridoside et d'acide iséthionique, dans un rapport massique compris entre 1 et 5, et un véhicule cosmétiquement acceptable, **caractérisée en ce que** l'extrait d'algue rouge comprend, en poids par rapport au poids total de la matière sèche :

- Floridoside 25% - 50%
- Acide iséthionique 10% - 25%

**2.** Composition cosmétique selon la revendication 1, **caractérisée en ce qu'**elle comprend en outre un extrait peptidique de spiruline.

**3.** Utilisation d'une composition cosmétique selon l'une quelconque des revendications précédentes, pour hydrater la peau et/ou pour prévenir le vieillissement cutané.

**4.** Méthode de traitement cosmétique des peaux déshydratées et/ou sèches, **caractérisée en ce qu'**elle comprend une étape d'application topique sur la peau d'une composition cosmétique selon l'une quelconque des revendications 1 ou 2.

**5.** Méthode de traitement cosmétique des peaux âgées et/ou ridées, **caractérisée en ce qu'**elle comprend une étape d'application topique sur la peau d'une composition cosmétique selon l'une quelconque des revendications 1 ou 2.

**6.** Extrait d'algue rouge, **caractérisé en ce qu'**il comprend, en poids par rapport au poids total de la matière sèche :

- Floridoside 25% - 50%
- Acide iséthionique 10% - 25%

**7.** Extrait selon la revendication 6, **caractérisé en ce que** l'algue rouge appartient au genre *Mastocarpus,* avantageusement à l'espèce *Mastocarpus stellatus.*

**8.** Extrait selon l'une quelconque des revendications 6 à 7, **caractérisé en ce qu'**il est obtenu par un procédé comprenant une étape d'ultrafiltration avec une membrane ayant un seuil de coupure de 10 000 à 20 000 Daltons suivie d'une étape de nanofiltration avec une membrane ayant un seuil de coupure de 100 à 200 Daltons.

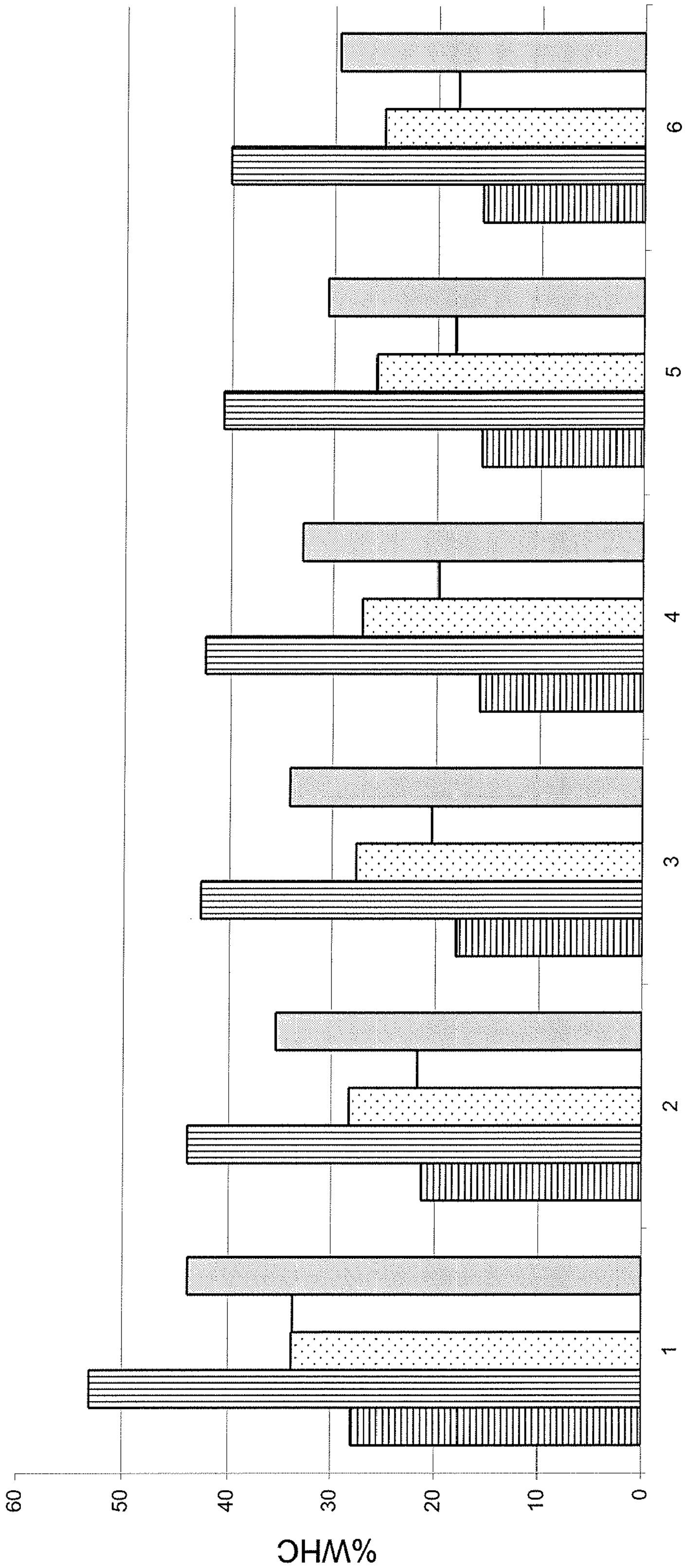


Fig. 1

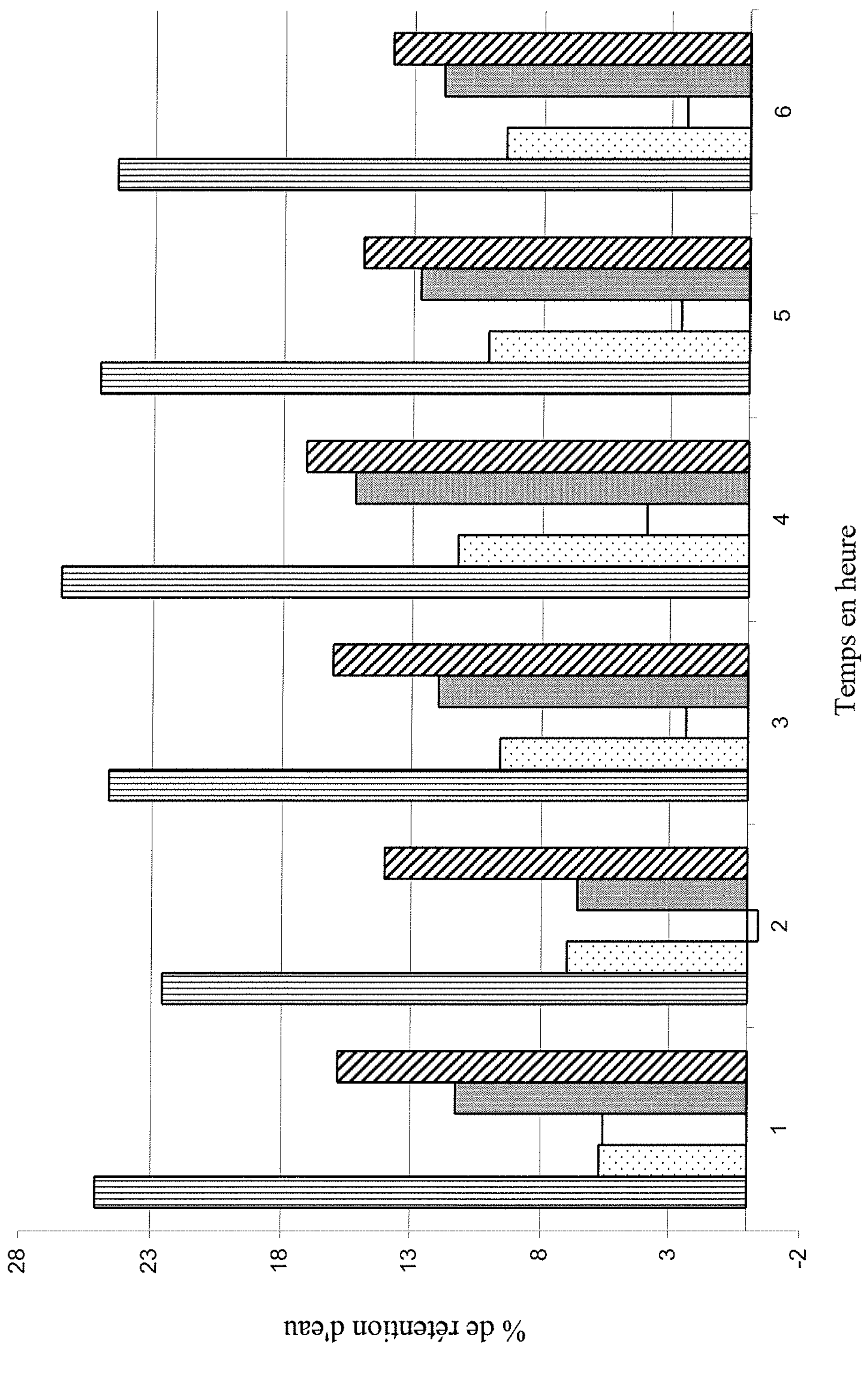


Fig. 2

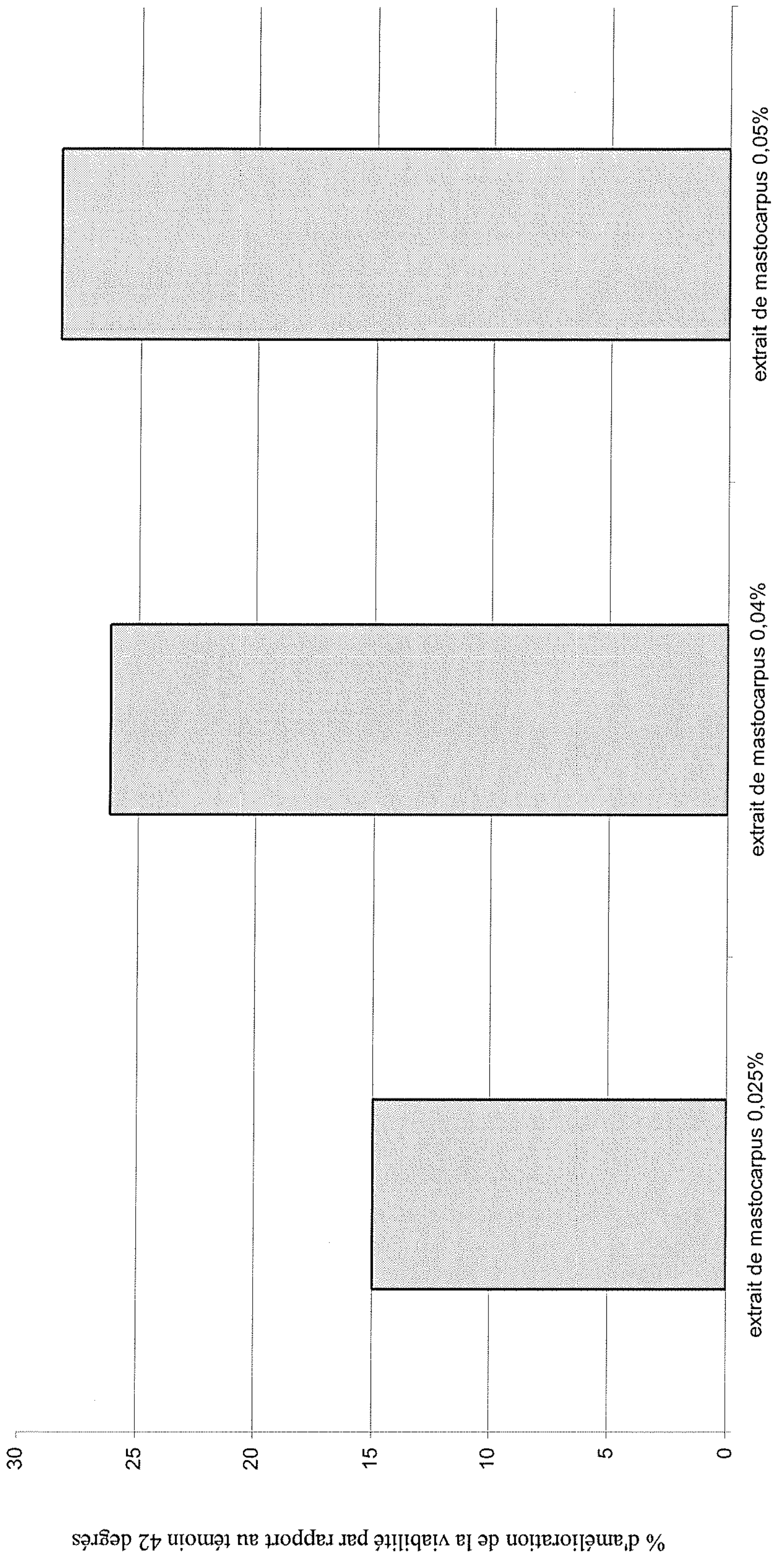
% d'amélioration de la viabilité par rapport au témoin 42 degrés
30
25
20
15
10
5
0
extrait de mastocarpus 0,025%
extrait de mastocarpus 0,04%
extrait de mastocarpus 0,05%

Fig. 3

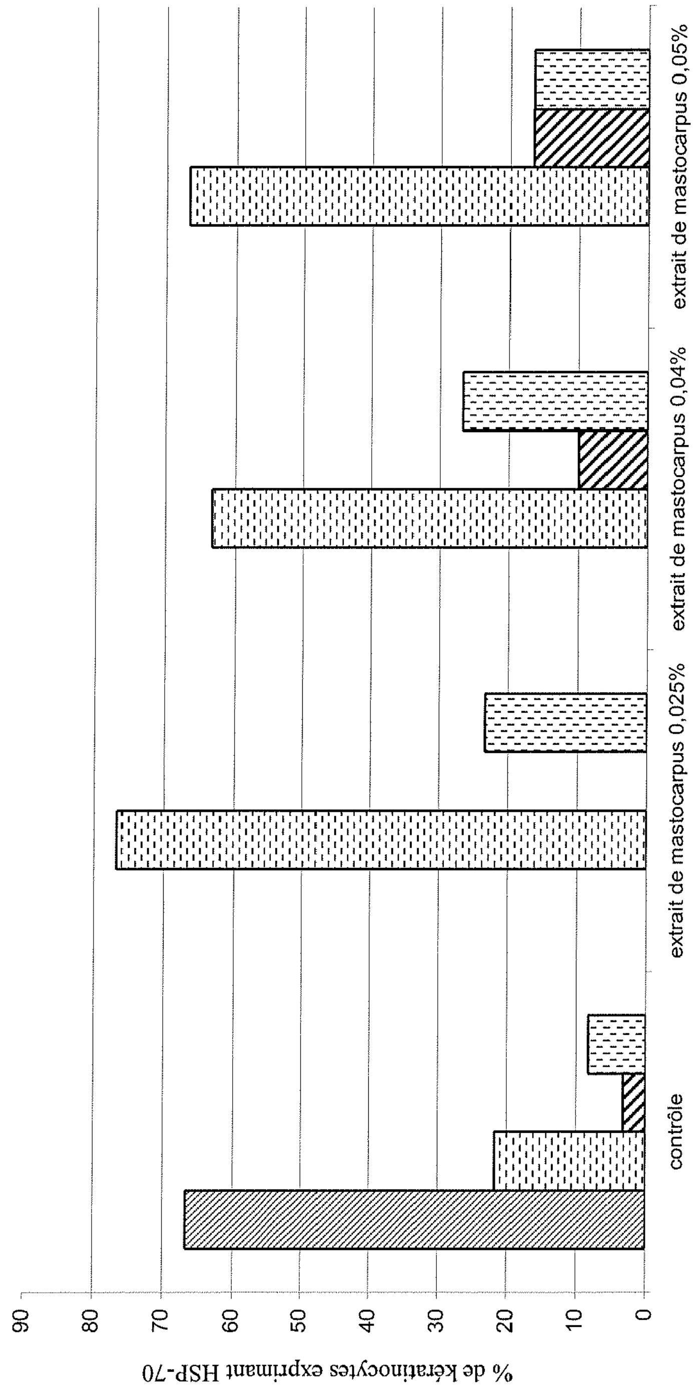
% de kératinocytes exprimant HSP-70
0
10
20
30
40
50
60
70
80
90
contrôle
extrait de mastocarpus 0,025%
extrait de mastocarpus 0,04%
extrait de mastocarpus 0,05%

Fig. 4

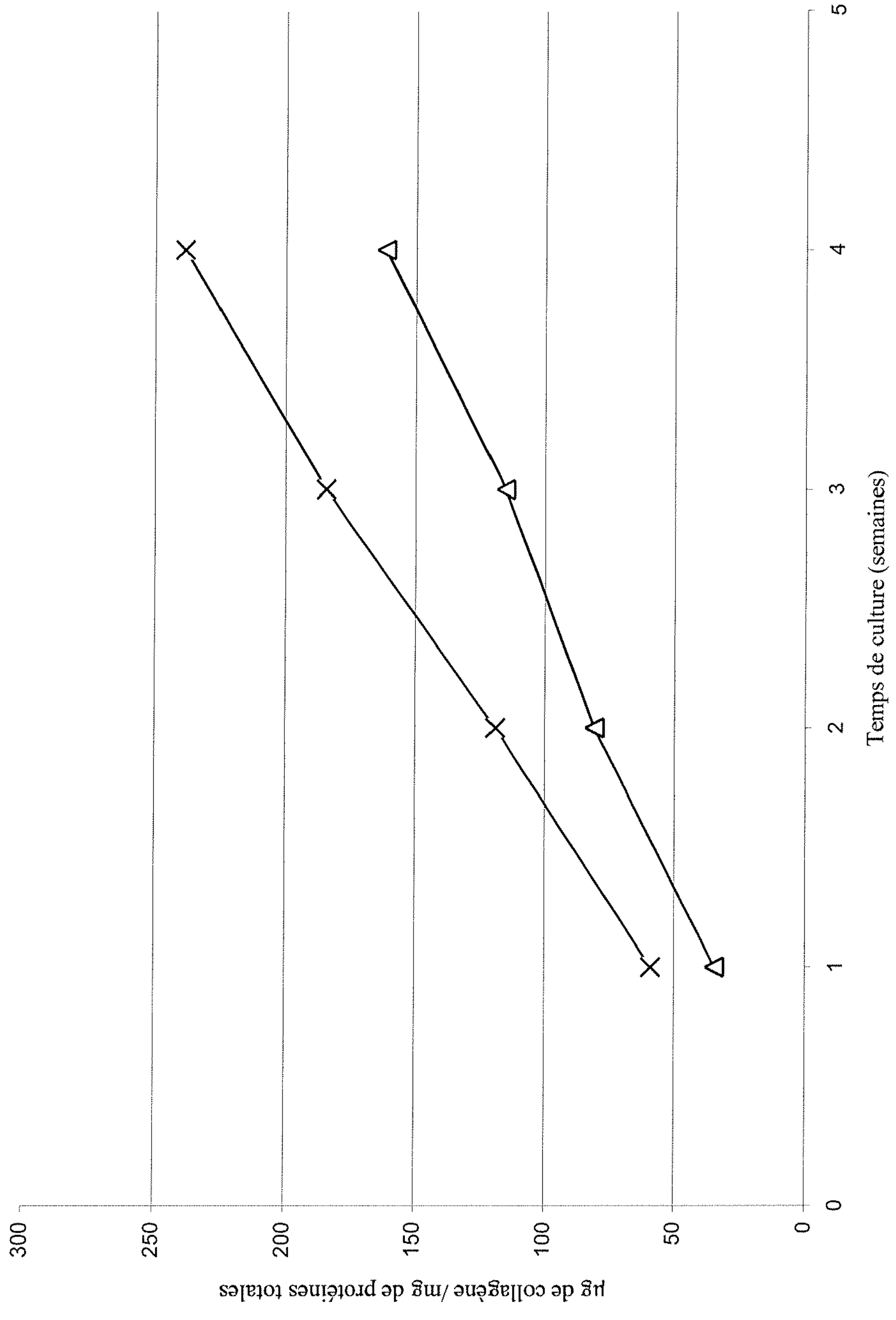
µg de collagène /mg de protéines totales
300
250
200
150
100
50
0
0
1
2
3
4
5
Temps de culture (semaines)

Fig. 5

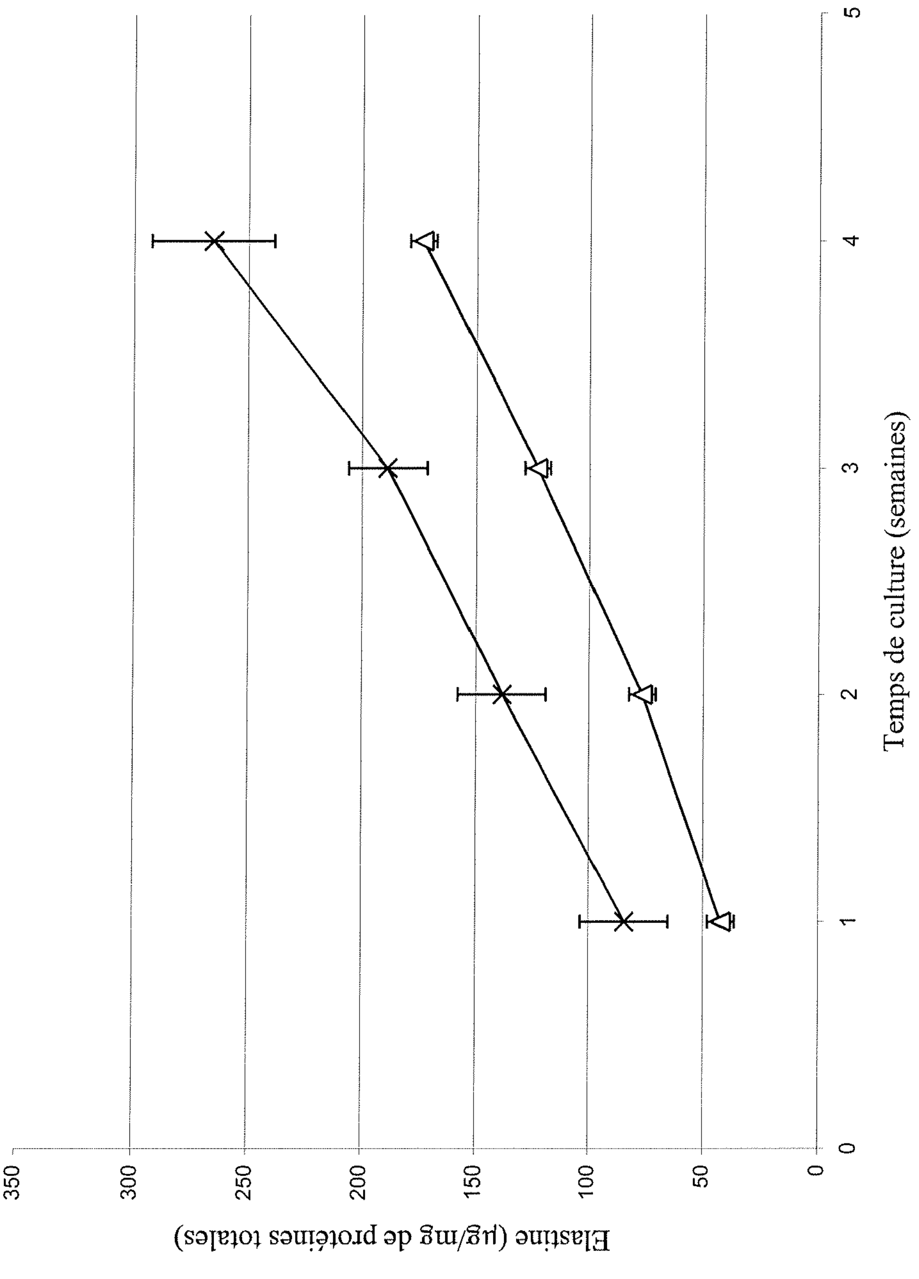
Elastine (µg/mg de protéines totales)
0
50
100
150
200
250
300
350
Temps de culture (semaines)
0
1
2
3
4
5

Fig. 6

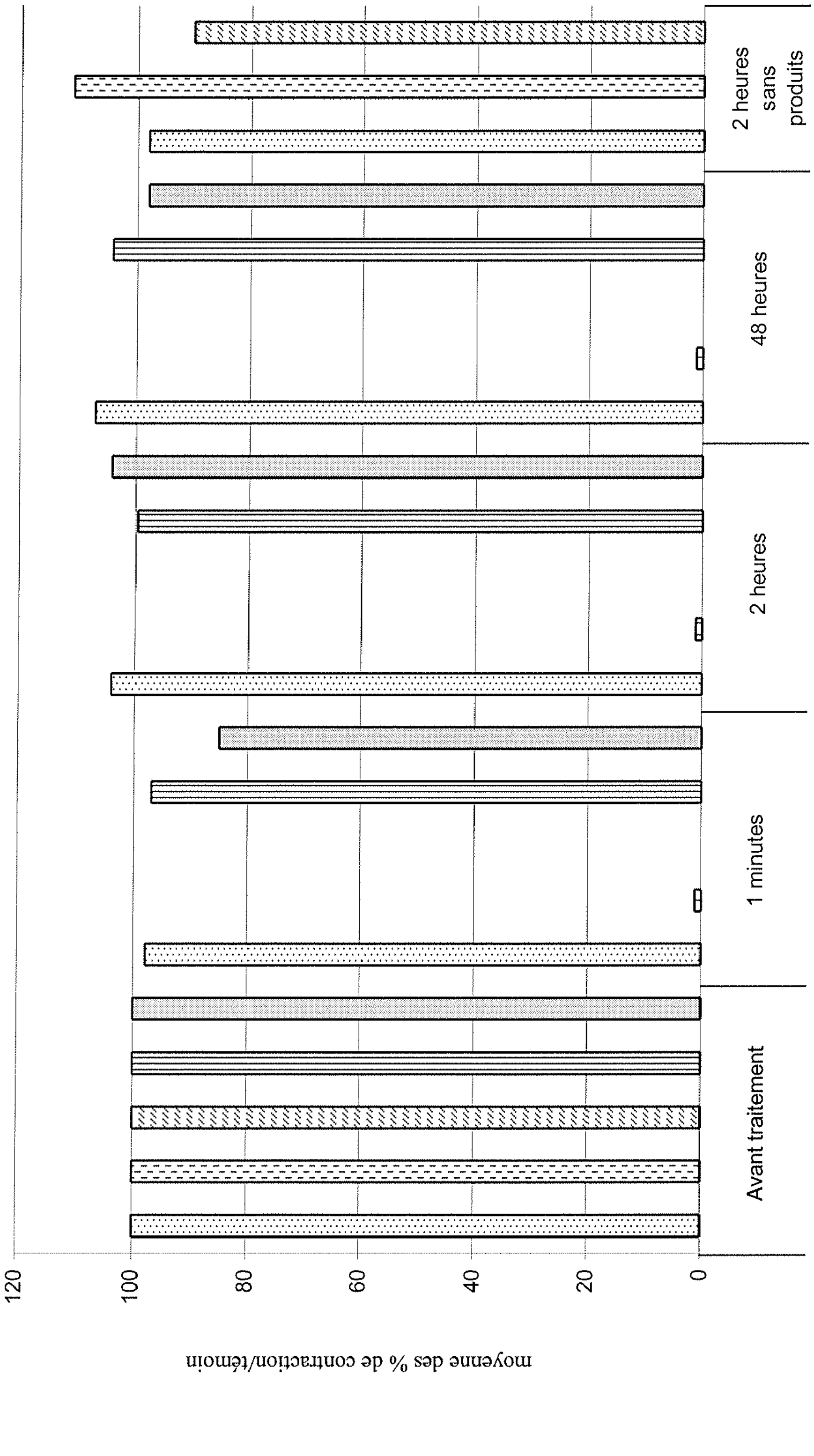
moyenne des % de contraction/témoin
120
100
80
60
40
20
0
Avant traitement
1 minutes
2 heures
48 heures
2 heures sans produits

Fig. 7

Office européen des brevets

# RAPPORT DE RECHERCHE EUROPEENNE

Numéro de la demande
EP 06 11 7159

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| X<br>Y | FR 2 655 268 A (SECMA)<br>7 juin 1991 (1991-06-07)<br>* page 1, ligne 28 - ligne 30; revendications 1,2,5,6 *<br>* page 7, ligne 5 - ligne 7 *<br>----- | 1,3-7<br>8 | INV.<br>A61K8/97 |
| A | US 2004/228875 A1 (LECLERC CHRISTIAN ET AL) 18 novembre 2004 (2004-11-18)<br>* revendications 1,8; exemples 5,7 *<br>----- | 1-6 | |
| X | DATABASE BIOSIS [Online]<br>BIOSCIENCES INFORMATION SERVICE, PHILADELPHIA, PA, US;<br>juillet 2003 (2003-07),<br>PEREIRA LEONEL ET AL: "Use of FTIR, FT-Raman and 13C-NMR spectroscopy for identification of some seaweed phycocolloids."<br>XP002376608<br>Database accession no. PREV200300515470<br>* abrégé *<br>& BIOMOLECULAR ENGINEERING,<br>vol. 20, no. 4-6, juillet 2003 (2003-07), pages 223-228,<br>ISSN: 1389-0344<br>----- | 6,7 | DOMAINES TECHNIQUES RECHERCHES (IPC)<br>A61K |
| Y | US 6 346 252 B1 (MOIGNE JEAN-YVES [FR])<br>12 février 2002 (2002-02-12)<br>* colonne 5, ligne 61 - colonne 6, ligne 17 *<br>* colonne 1, ligne 18 - ligne 26 *<br>----- | 8 | |

Le présent rapport a été établi pour toutes les revendications

| Lieu de la recherche | Date d'achèvement de la recherche | Examinateur |
|---|---|---|
| Berlin | 1 novembre 2006 | Angiolini, Delia |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)

**ANNEXE AU RAPPORT DE RECHERCHE EUROPEENNE**
**RELATIF A LA DEMANDE DE BREVET EUROPEEN NO.** EP 06 11 7159

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

01-11-2006

| Document brevet cité au rapport de recherche | | Date de publication | Membre(s) de la famille de brevet(s) | | | Date de publication |
|---|---|---|---|---|---|---|
| FR 2655268 | A | 07-06-1991 | DE | 69007626 | D1 | 28-04-1994 |
| | | | DE | 69007626 | T2 | 25-08-1994 |
| | | | EP | 0504236 | A1 | 23-09-1992 |
| | | | ES | 2054485 | T3 | 01-08-1994 |
| | | | WO | 9107946 | A1 | 13-06-1991 |
| | | | JP | 5504583 | T | 15-07-1993 |
| US 2004228875 | A1 | 18-11-2004 | AU | 2002352337 | A1 | 26-05-2003 |
| | | | CA | 2467102 | A1 | 22-05-2003 |
| | | | CN | 1617702 | A | 18-05-2005 |
| | | | EP | 1443898 | A2 | 11-08-2004 |
| | | | WO | 03041679 | A2 | 22-05-2003 |
| | | | FR | 2832629 | A1 | 30-05-2003 |
| | | | JP | 2005508999 | T | 07-04-2005 |
| US 6346252 | B1 | 12-02-2002 | AT | 194262 | T | 15-07-2000 |
| | | | DE | 69702455 | D1 | 10-08-2000 |
| | | | DE | 69702455 | T2 | 08-03-2001 |
| | | | EP | 0926956 | A1 | 07-07-1999 |
| | | | ES | 2150275 | T3 | 16-11-2000 |
| | | | FR | 2753101 | A1 | 13-03-1998 |
| | | | WO | 9810656 | A1 | 19-03-1998 |
| | | | JP | 2001503027 | T | 06-03-2001 |

EPO FORM P0460

Pour tout renseignement concernant cette annexe : voir Journal Officiel de l'Office européen des brevets, No.12/82

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- FR 2655268
- WO 03041679 A
- FR 2857978